# EUROPÄISCHE PATENTSCHRIFT

(11) **EP 0 710 882 B1**
(45) Veröffentlichungstag und Bekanntmachung des Hinweises auf die Patenterteilung: **03.01.2001**
(21) Anmeldenummer: 95116656.0
(22) Anmeldetag: 23.10.1995
(51) Int. Cl.: G03C 7/38, C07D 471/04

(54) **Farbfotografisches Aufzeichnungsmaterial**
Colour photographic material
Matériau photographique couleur

(30) Priorität: 04.11.1994 DE 4439329
(43) Veröffentlichungstag der Anmeldung: 08.05.1996
(73) Patentinhaber: AGFA-GEVAERT naamloze vennootschap, 2640 Mortsel (BE)
(72) Erfinder: Schenk, Günther, Dr., D-51467 Bergisch Gladbach (DE)

(56) Entgegenhaltungen:
- EP-A- 0 376 166

## Beschreibung

Die Erfindung betrifft ein fotografisches Farbentwicklungsverfahren, bei dem ein farbfotografisches Aufzeichnungsmaterial mit mindestens einer bildmäßig belichteten Silberhalogenidemulsionsschicht in Gegenwart einer bestimmten Farbkupplerverbindung und einer Farbentwicklerverbindung entwickelt wird, und ein farbfotografisches Aufzeichnungsmaterial, das bestimmte Farbkuppler enthält. Bei den erfindungsgemäß verwendeten Farbkupplem handelt es sich um Verbindungen der folgenden allgemeinen Formel I 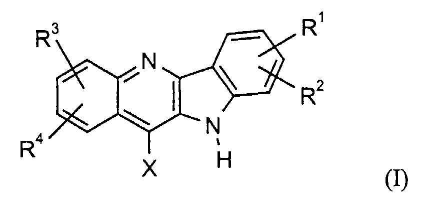 worin
- R¹, R², R³, R⁴: ein H-Atom oder einen Substituenten;
- X: ein H-Atom oder einen Rest, der unter den Bedingungen der chromogenen Entwicklung abgespalten wird, bedeuten.

Es ist bekannt, farbige fotografische Bilder durch chromogene Entwicklung herzustellen, d.h. dadurch, daß man bildmäßig belichtete Silberhalogenidemulsionsschichten in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere vom p-Phenylendiamintyp, verwendet.

An die Farbkuppler sowie an die daraus durch chromogene Entwicklung erhaltenen Farbstoffe wird in der Praxis eine Reihe von Anforderungen gestellt. So soll die Kupplungsgeschwindigkeit der Farbkuppler mit dem Oxidationsprodukt des Farbentwicklers möglichst groß sein. Die Farbkuppler, sowie die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material. Beispielsweise darf der in den Bildweißen des verarbeiteten Materials noch vorhandene restliche Kuppler nicht vergilben. Außerdem sollen die Farbstoffe hinreichend beständig sein gegenüber gasförmigen reduzierenden oder oxidierenden Agentien. Sie müssen ferner diffusionsfest verankert sein und sollen sich bei der chromogenen Entwicklung als möglichst feines Korn abscheiden. Die mechanischen Eigenschaften der Schichten dürfen durch die Farbkuppler nicht beeinträchtigt werden. Schließlich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen. So soll ein Gelbfarbstoff im Idealfall blaues Licht absorbieren und grünes sowie rotes Licht weitgehend durchlassen. Außerdem sollen die Absorptionsmaxima der Farbstoffe sowohl bei Colorumkehr- wie auch bei Colornegativfilmen möglichst mit dem Sensibilisierungsmaxima der zum Kopieren verwendeten Colorpapiermaterialien übereinstimmen.

R¹, R², R³, R⁴, mindestens jedoch einer dieser Substituenten, können zur Ballastierung der Kuppler dienen.

Aufzeichnungsmaterialien in denen, der Farbkuppler der Formel II entspricht 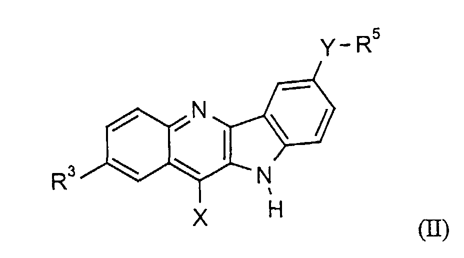 worin
- R³: H oder Alkyl;
- R⁵: eine gegebenenfalls substituierte Alkylgruppe;
- X: H oder einen unter den Bedingungen der chromogenen Entwicklung abspaltbaren Rest;
- Y: -NH-CO- oder -NH-SO₂- bedeuten,
sind besonders bevorzugt.

Es gibt keine spezielle Einschränkung für die Substituenten R¹, R², R³ und R⁴, typische Beispiele sind z.B. Halogen, Alkyl, Cycloalkyl, Aryl, Anilino, Acylamino, Sulfonamido, Alkylthio, Arylthio, Alkenyl, ferner Cycloalkenyl, Alkinyl, Heterocyclyl, Sulfonyl, Sulfinyl, Phosphonyl, Acyl, Carbamoyl, Sulfamoyl, Cyano, Alkoxy, Sulfonyloxy, Aryloxy, Heterocyclyloxy, Siloxy, Acyloxy, Carbamoyloxy, Amino, Alkylamino, Imido, Ureido, Sulfamoylamino, Alkoxycarbonylamino, Aryloxycarbonylamino, Alkoxycarbonyl, Aryloxycarbonyl, Heterocyclylthio, Thioureido, Carboxy, Nitro, Sulfonat, usw. Spirosubstituenten, sowie Brückenkohlenwasserstoffreste.

Alkyl-Sustituenten für R¹, R², R³ bzw. R⁴ sind hier vorzugsweise Reste mit 1 bis 32 C-Atomen, geradkettig oder verzweigt, unsubstituiert oder substituiert, z.B. substituiert mit Alkoxy, Aryloxy oder Acyl.

Aryl-Substituenten für R¹, R², R³ bzw. R⁴ sind hier vorzugsweise Phenyl bzw. substituierte Phenylreste.

Acylamino-Substituenten für R¹, R², R³ bzw. R⁴ sind hier bevorzugt Alkylcarbonylamino, Arylcarbonylamino, Hetarylcarbonylamino, Ureido.

Sulfonamido-Substituenten für R¹, R², R³ bzw. R⁴ sind hier bevorzugt Alkylsulfonylamino, Arylsulfonylamino, Hetarylsulfonylamino.

Bei den Alkylthio- und Arylthio-Substituenten für R¹, R², R³ bzw. R⁴ sind die Alkyl- bzw. Aryl-Fragmente in ihrer Bandbreite identisch mit der Beschreibung der Substituenten Alkyl und Aryl R¹, R², R³ bzw. R⁴.

Alkenyl-Substituenten für R¹, R², R³ bzw. R⁴ enthalten 2 bis 32 Kohlenstoffatome; der Alkenyl-Substituent kann geradkettig oder verzweigt sein.

Cycloalkyl- und Cycloalkenyl-Substituenten für R¹, R², R³ bzw. R⁴ enthalten 3 bis 12 C-Atome, vorzugsweise 5 bis 7 C-Atome.

Sulfonyl-Substituenten für R¹, R², R³ bzw. R⁴ sind z.B. jede Art Alkylsulfonyl, Arylsulfonyl oder Hetarylsulfonyl.

Sulfinyl-Substituenten für R¹, R², R³ bzw. R⁴ sind z.B. jede Art Alkylsulfinyl, Arylsulfinyl oder Hetarylsulfinyl.

Phosphonyl-Substituenten für R¹, R², R³ bzw. R⁴ sind z.B. jeder Art Alkylphosphonyl, Alkoxyphosphonyl, Aryloxyphosphonyl oder Arylphosphonyl.

Acyl-Substituenten für R¹, R², R³ bzw. R⁴ sind z.B. jede Art Alkylcarbonyl oder Arylcarbonyl.

Carbamoyl-Substituenten für R¹, R², R³ bzw. R⁴ sind z.B. jede Art Alkylcarbamoyl, Arylcarbamoyl oder Hetarylcarbamoyl.

Sulfamoyl-Substituenten für R¹, R², R³ bzw. R⁴ sind z.B. jede Art Alkylsulfamoyl, Arylsulfamoyl oder Hetarylsulfamoyl.

Acyloxy-Substituenten für R¹, R², R³ bzw. R⁴ sind z.B. jede Art Alkylcarbonyloxy oder Arylcarbonyloxy.

Carbamoyloxy-Substituenten für R¹, R², R³ bzw. R⁴ sind z.B. jede Art Alkylcarbamoyloxy oder Arylcarbamoyloxy.

Ureido-Substituenten für R¹, R², R³ bzw. R⁴ sind z.B. jede Art Alkylureido, Arylureido oder Hetarylureido.

Sulfamoylamino-Substituenten für R¹, R², R³ bzw. R⁴ sind z.B. jede Art von Alkylsulfamoylamino oder Arylsulfamoylamino.

Heterocyclische Substituenten für R¹, R², R³ bzw. R⁴ sind vorzugsweise 5- bis 7-Ringheterocyclen; typische Beispiele sind: 2-Furyl, 2-Thienyl, 2-Pyrimidinyl, 2-Benzthiazolyl, 1-Pyrrolyl, 1-Tetrazolyl.

Heterocyclyloxy-Substituenten für R¹, R², R³ bzw. R⁴ sind vorzugsweise über ein O-Atom gebundene 5- bis 7-Ringheterocyclen; typische Beispiele sind: 3,4,5,6-Tetrahydropyranyl-2-oxy, 1-Phenyltetrazol-5-oxy.

Heterocyclylthio-Substituenten für R¹, R², R³ bzw. R⁴ sind vorzugsweise über ein S-Atom gebundene 5- bis 7-Ringheterocyclen; typische Beispiele sind: 2-Pyridylthio, 2-Benzthiazolylthio, 2,4-Diphenoxy-1,3,5-triazin-6-yl-thio.

Imido-Substituenten für R¹, R², R³ bzw. R⁴ sind z.B. Succinimido, 3-Heptadecylsuccinimido, Phthalimido, Glutarimido.

Spiro-Substituenten für R¹, R², R³ bzw. R⁴ sind z.B. Spiro[3,3]heptan-1-yl.

Verbrückte Kohlenwasserstoff-Substituenten für R¹, R², R³ bzw. R⁴ sind z.B. Bicyclo[2,2,1]heptan-1-yl, Tricyclo[3,2,1,1]decan-1-yl, 7,7-Dimethyl-bicyclo[2,2,1]heptan-1-yl.

Ein durch X dargestellter, unter den Bedingungen der chromogenen Entwicklung abspaltbarer Rest, kann beispielsweise sein: H, ein Halogenatom, wie Cl, Br oder F, Substituenten wie: Alkoxy, Aryloxy, Heterocyclyloxy, Acyloxy, Sulfonyloxy, Alkoxycarbonyloxy, Aryloxycarbonyloxy, Alkyloxalyloxy, Alkoxyoxalyloxy, Alkylthio, Arylthio, Heterocyclylthio, Alkyloxythiocarbonylthio, Acylamino, Sulfonamido, ein N-haltiger Heterocyclus, der über ein N-Atom mit dem Kupplerrest verknüpft ist, Alkoxycarbonylamino, Aryloxycarbonylamino, Carboxyl, -N=N-Aryl.

Je nach dem Substituenten X an der zur Kupplung befähigten Position kuppeln die erfindungsgemäßen Cyankuppler entweder nach dem 4-Äquivalentprinzip oder als 2-Äquivalentkuppler, wobei der abgespaltene nukleophile Rest spezielle fotografische Wirkungen hervorrufen kann. Es kann zudem ein nukleophiler Rest abgespalten werden, der seine fotografische Wirkung erst nach Abspaltung eines Zwischengliedes entfaltet.

Eine in Formel I und Formel II durch X dargestellte unter den Bedingungen der chromogenen Entwicklung abspaltbare Gruppe ist beispielsweise entweder selbst der Rest einer fotografisch wirksamen Verbindung oder eine Gruppe, die nach Abspaltung aus der Kupplungsstelle des Kupplers bei dessen Kupplung mit dem Oxidationsprodukt des Silberhalogenidentwicklungsmittels befähigt ist, erst in einer Folgereaktion einen daran gebundenen Rest einer fotografisch wirksamen Verbindung freizusetzen. Eine solche Gruppe wird auch als Zeitsteuerglied bezeichnet, weil der daran gebundene Rest der fotografisch wirksamen Verbindung in vielen Fällen verzögert freigesetzt wird und erst dann wirksam werden kann. Bekannte Zeitsteuerglieder sind beispielsweise eine Gruppe 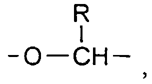 wobei das O-Atom an die Kupplungsstelle des Kupplers und das C-Atom an ein N-Atom einer fotografisch wirksamen Verbindung gebunden ist (z.B. DE-A-28 03 145), eine Gruppe, die nach Abspaltung vom Kuppler einer intramolekularen nukleophilen Verdrängungsreaktion unterliegt und hierbei die fotografisch wirksame Verbindung freisetzt (z.B. DE-A-28 55 697), eine Gruppe, in der nach Abspaltung vom Kuppler eine Elektronenübertragung entlang eines konjugierten Systems stattfinden kann, wodurch die fotografische wirksame Verbindung freigesetzt wird (z.B. DE-A-31 05 026), oder eine Gruppe 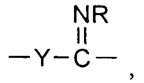 worin Y (z.B. -O-) an die Kupplungsstelle des Kupplers und das C-Atom an ein Atom der fotografisch wirksamen Verbindung gebunden ist und worin R beispielsweise für Aryl steht (z.B. EP-A-0 127 063). Das Zeitsteuerglied kann auch eine Gruppe sein, die nach Abspaltung aus der Kupplungsstelle des Kupplers selbst eine Kupplungsreaktion oder eine Redoxreaktion eingehen kann und als Folge einer solchen Reaktion die an sie gebundene fotografisch wirksame Verbindung freisetzt.

Bei der unter den Bedingungen der chromogenen Entwicklung abspaltbaren Gruppe handelt es sich beispielsweise um eine organische Gruppe, die in der Regel über ein Sauerstoff-, Schwefel- oder Stickstoffatom an die Kupplungsstelle des Kupplermoleküls angeknüpft ist. Falls es sich bei der abspaltbaren Gruppe um eine cyclische Gruppe handelt, kann die Anknüpfung an die Kupplungsstelle des Kupplermoleküls entweder direkt über ein Atom, das Bestandteil eines Ringes ist, z.B. ein Stickstoffatom, oder indirekt über ein zwischengeschaltetes Bindeglied erfolgt sein. Derartige abspaltbare Gruppen sind in großer Zahl bekannt, z.B. als Fluchtgruppen von 2-Äquivalentkupplern.

Beispiele von über Sauerstoff angeknüpften abspaltbaren Gruppen entsprechen der Formel

-O-R^{5a},

worin R^{5a} für einen acyclischen oder cyclischen organischen Rest steht, z.B. für Alkyl, Aryl, eine heterocyclische Gruppe oder Acyl, das sich beispielsweise ableitet von einer organischen Carbon- oder Sulfonsäure.

Bei besonders bevorzugten abspaltbaren Gruppen dieser Art bedeutet R⁵ eine gegebenenfalls substituierte Phenylgruppe. Solche Gruppen sind beispielsweise beschrieben in US-A-3 408 194, DE-A-24 56 076.

Beispiele von über Stickstoff angeknüpften abspaltbaren Gruppen sind in den folgenden deutschen Offenlegungsschriften (DE-A-) beschrieben:
20 57 941, 21 63 812, 22 13 461, 22 19 917, 22 61 361, 22 63 875, 23 18 807, 23 29 587, 23 44 155, 23 63 675, 24 33 812, 24 41 779, 24 42 703, 25 28 638, 25 28 860, 26 37 817, 28 18 373, 30 20 416.

Hierbei handelt es sich durchweg um 5- oder 6-gliedrige heterocyclische Ringe, die über ein Ringstickstoffatom mit der Kupplungsstelle des Kupplers verbunden sind. Die heterocyclischen Ringe enthalten vielfach benachbart zu dem die Bindung an das Kupplermolekül vermittelnden Stickstoffatom aktivierende Gruppen, z.B. Carbonyl- oder Sulfonylgruppen oder Doppelbindungen.

Beispielhaft für solche unter den Bedingungen der chromogenen Entwicklung abspaltbaren Reste sind im folgenden angegeben.

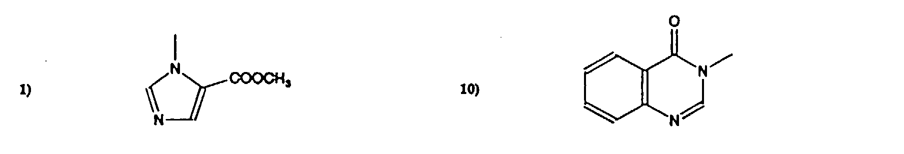

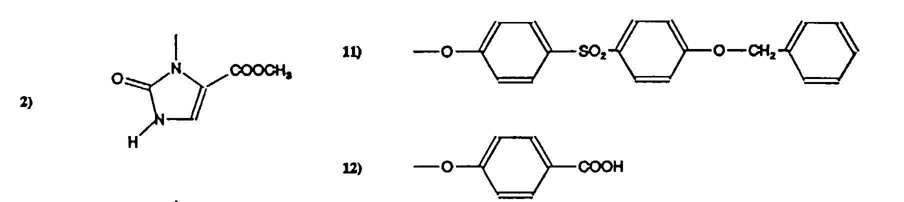

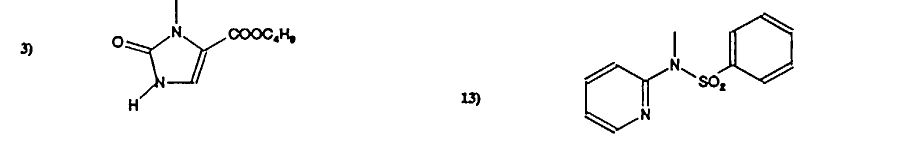

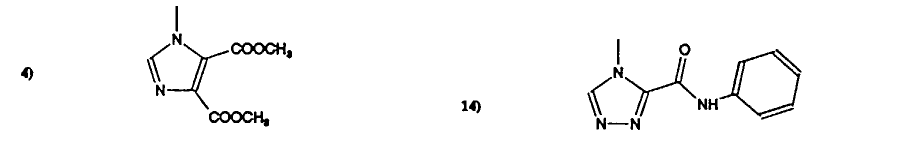

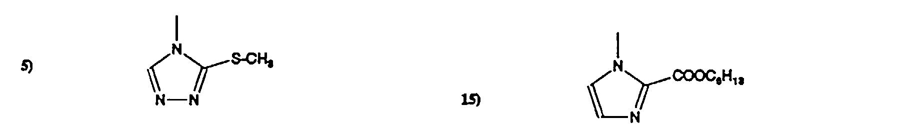

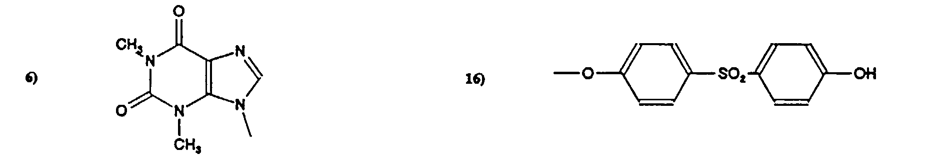

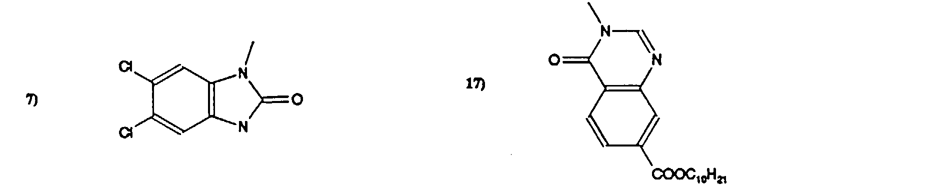

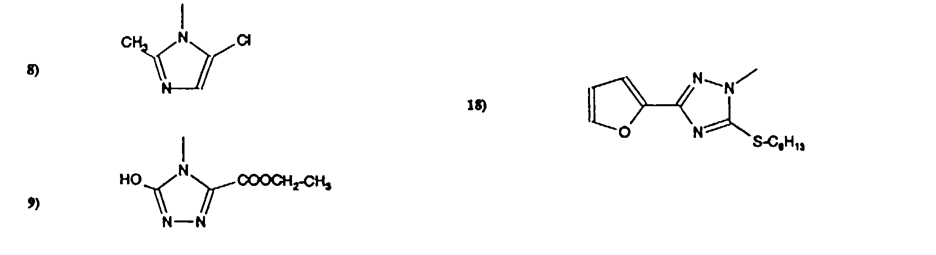

Bei der fotografisch wirksamen Verbindung kann es sich um einen Entwicklungsinhibitor, einen Bleichinhibitor, einen Entwicklungsbeschleuniger, ein silberkeimbildendes "Nukleierungsmittel", eine lösliche ausentwicklungsfördernde Mercaptanverbindung, einen Stabilisator, einen Weißkuppler, einen Scavanger, ein Elektronentransferagens, z.B. vom Phenidontyp oder eine Farbkupplerverbindung handeln.

Als Entwicklungsinhibitoren sind insbesondere solche aus der Reihe der Benzotriazole, der Thienotriazole, der nichtkondensierten monocyclischen 1,2,3-Triazole, der 3-Alkylthio-1,2,4-triazole, der 5-Mercapto-1-alkyl- oder 5-Mercapto-1-aryltetrazole sowie der 2-Mercapto-5-alkylthio-1,3,4-thiadiazole zu nennen.

Die erfindungsgemäßen Farbkuppler können weiterhin mit einem Ballastrest R¹, R², R³, R⁴ ausgestattet sein. Als Ballastreste sind solche Reste anzusehen, die es ermöglichen oder erleichtern, die erfindungsgemäßen Farbkuppler in den üblicherweise bei fotographischen Materialien verwendeten hydrophilen Kolloiden diffusionsfest einzulagern. Hierzu sind vozugsweise organische Reste geeignet, die im allgemeinen geradkettige oder verzweigte aliphatische Gruppen und gegebenenfalls auch carbocyclische oder heterocyclische aromatische Gruppen mit im allgemeinen 8 - 20 C-Atome enthalten.

Beispiele für erfindungsgemäße Farbkuppler sind im folgenden aufgeführt.

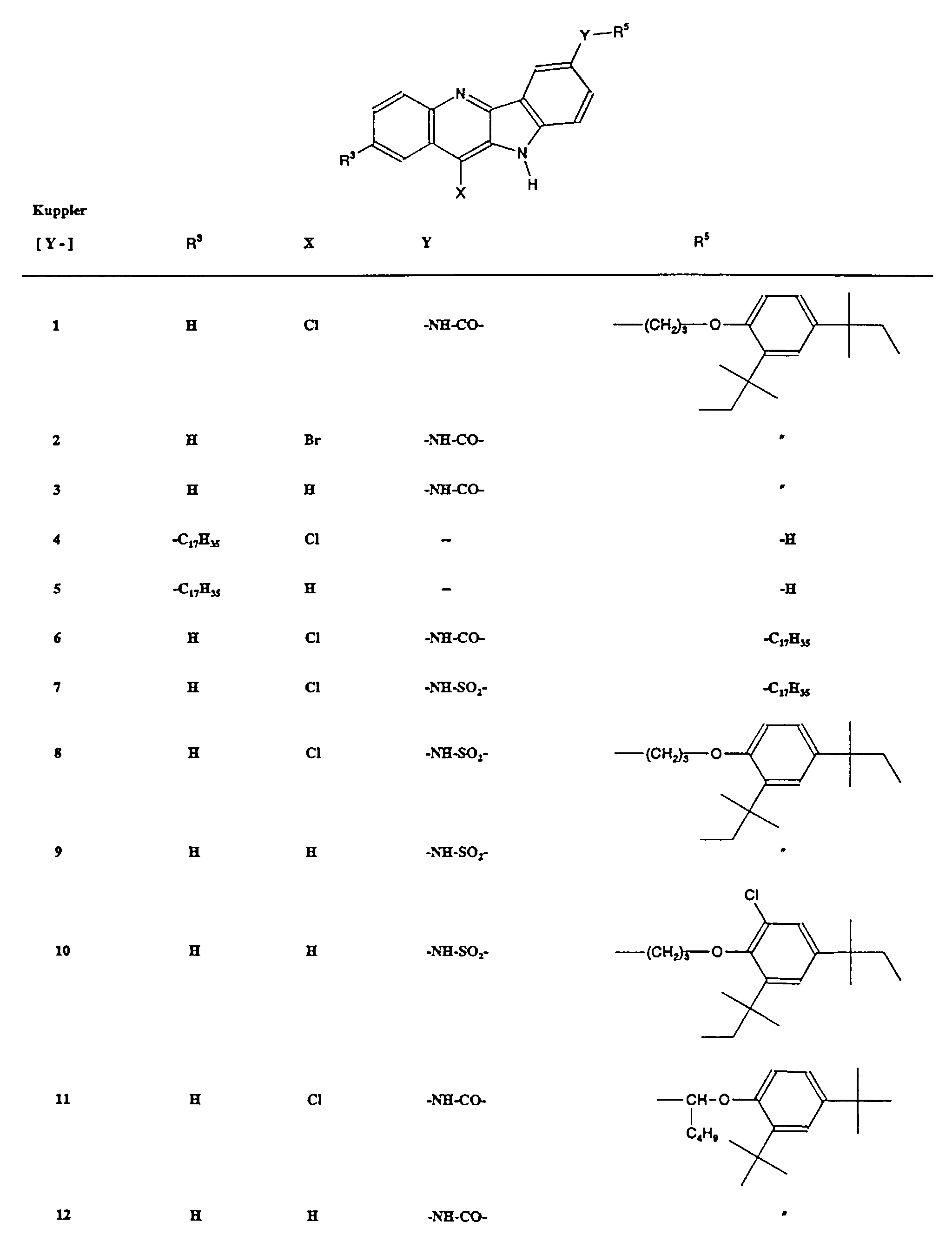

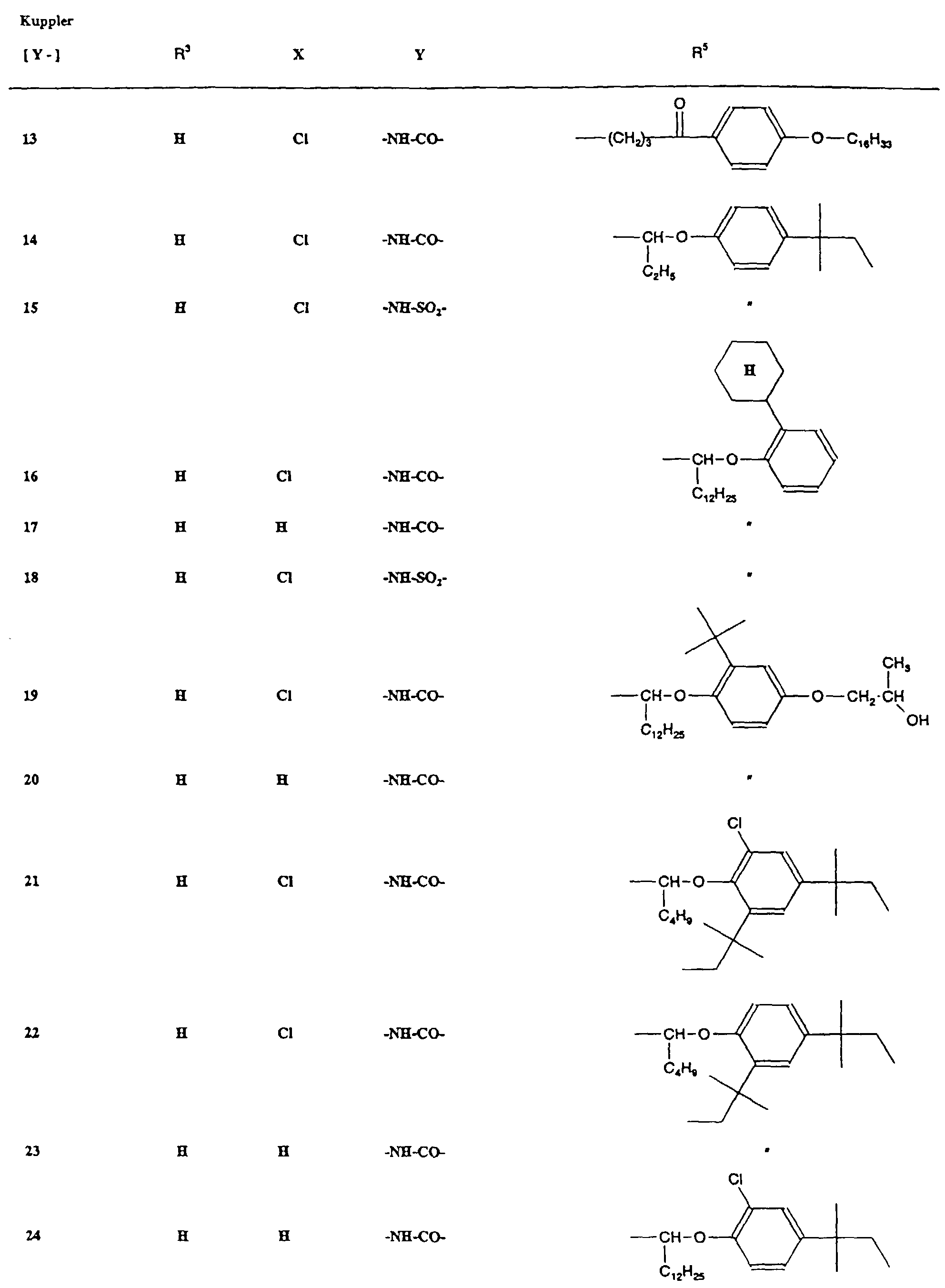

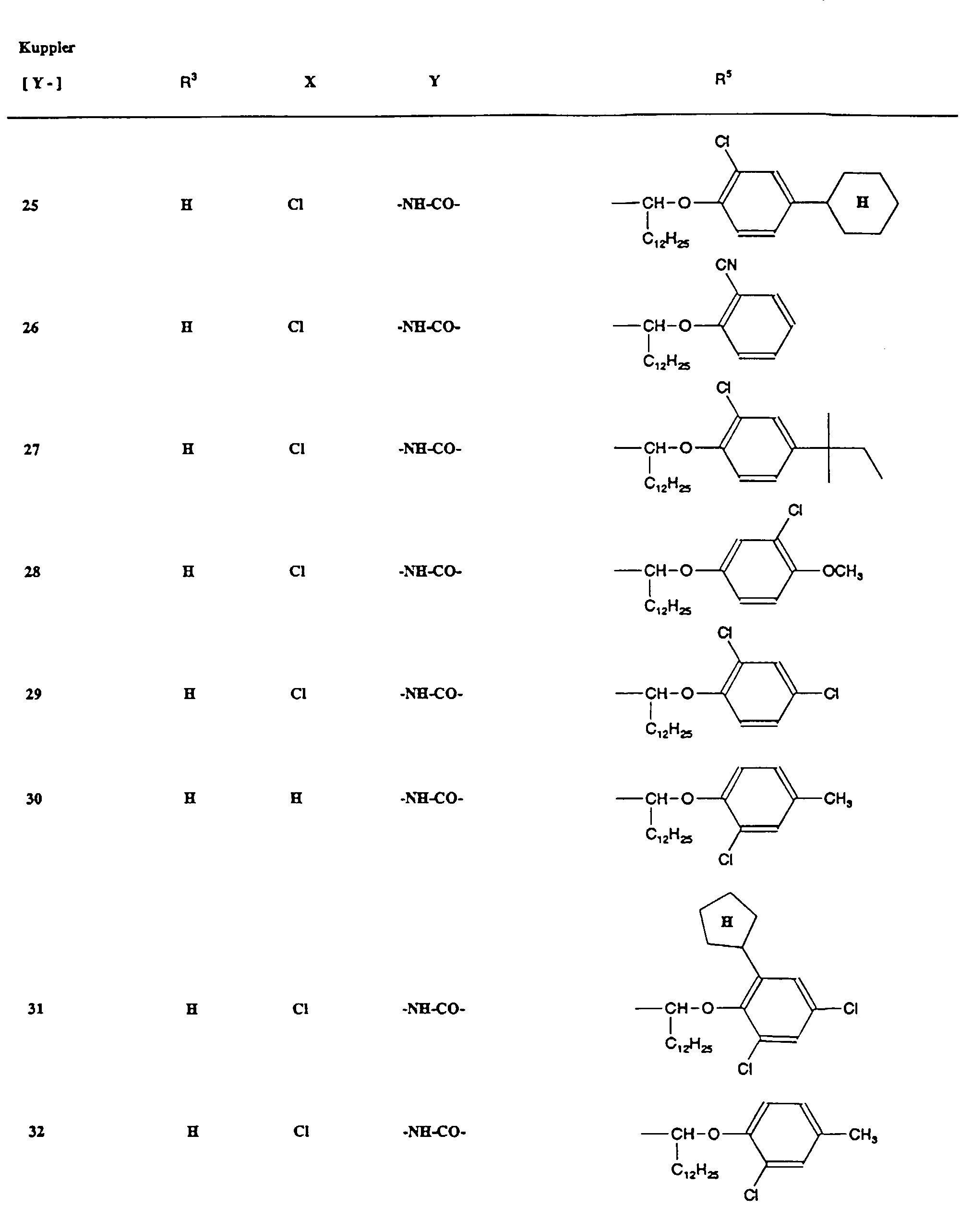

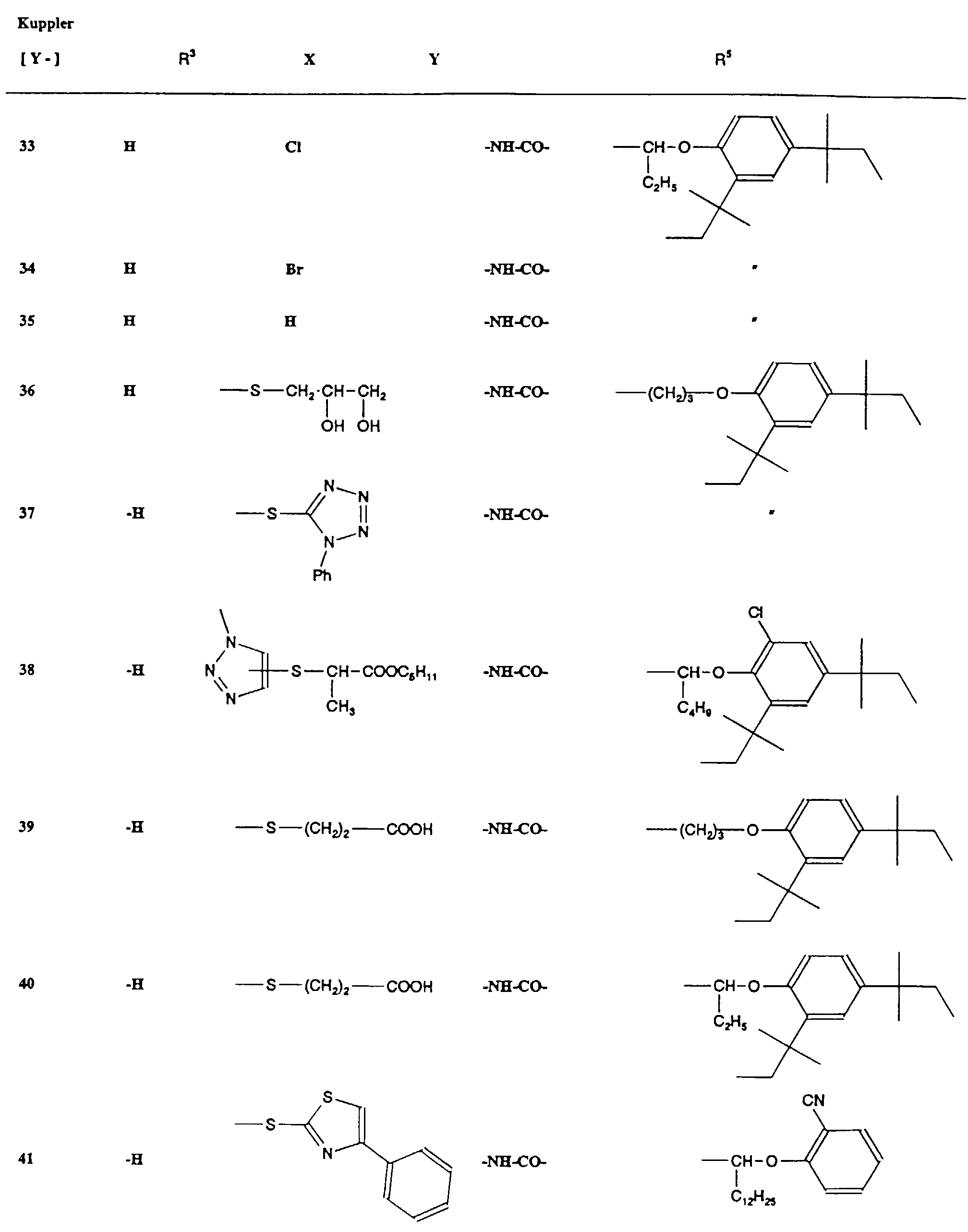

Indolochinoline sind als organ. Verbindungen wenig beschrieben (speziell derivatisiert als potentielle Antitumor-Wirkstoffklasse, s. z.B. EP-A 0 376 166), nicht jedoch als fotografische Kupplungskomponenten zur Erzeugung von gelben Farbstoffchromophoren.

Die erfindungsgemäßen Farbkuppler können u. a. nach folgendem allgemeinen Prinzip hergestellt werden:

Cyclisierung von 4-Hydroxychinolinen, bzw. deren tautomeren 4-Chinolinonen mit Anilinen in Polyphosphorsäure zu 5H,10H-indolo-[3,2-b]chinolin-11-onen, welche mit Phoshoroxytrihalogenid zu den 11-Halogeno-indolo[3,2-b]chinolinen führen. Letztere können dann electrophil weitersubstituiert, oder auch enthalogeniert oder in Position 11 gegen Substituenten X ausgetauscht werden. Elektrophile Substitution in Form einer Nitrierung führt nach Reduktion zu z.B. 7-Amino-Derivaten, die dann z.B. mit "Ballastrest-Säurechloriden" ballastierte Farbkuppler ergeben, die eine diffusionsfeste Einlagerung in fotografische Materialien ermöglichen.

Die Synthese kann z.B. nach dem nachfolgenden allgemeinen Schema erfolgen.

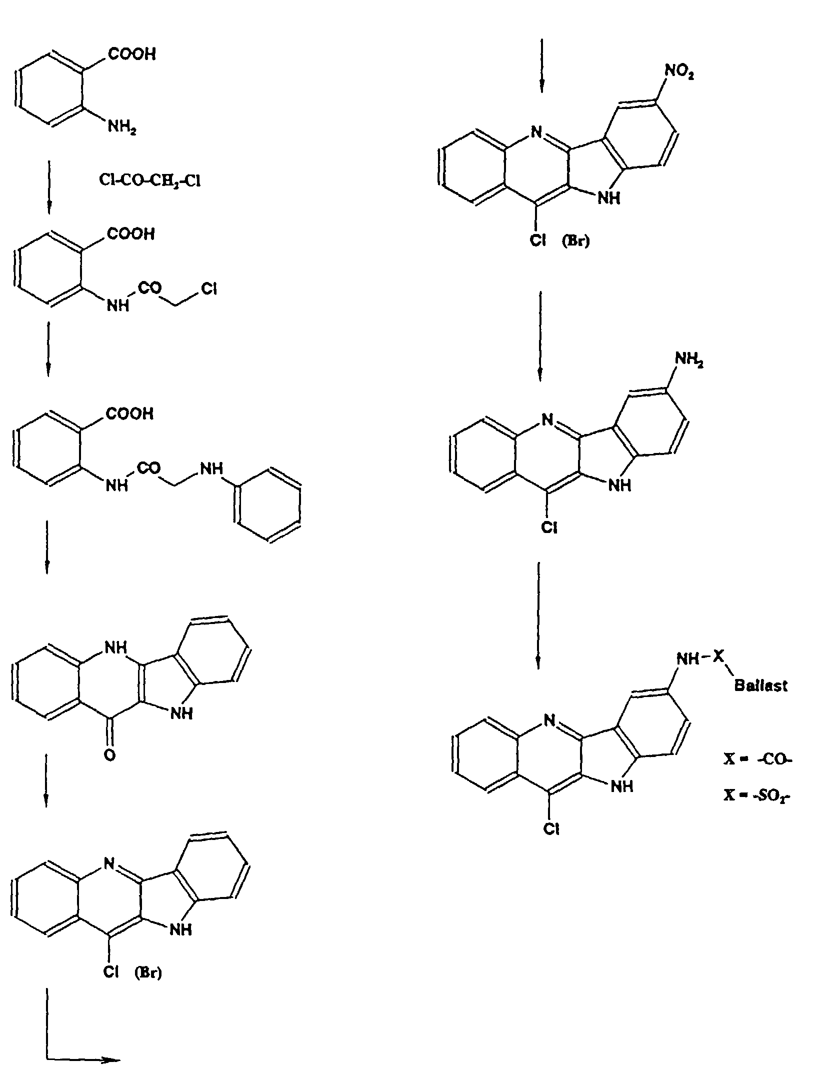

Bei dem erfindungsgemäßen Verfahren wird ein farbfotografisches Material, das wenigstens eine bildmäßig belichtete Silberhalogenidemulsion enthält, mit einer Farbentwicklerverbindung vom p-Phenylendiamintyp entwickelt. Die erfindungsgemäßen Farbkuppler können dabei in räumlicher und spektraler Zuordnung zu einer lichtempfindlichen Silberhalogenidemulsionsschicht in dem Material enthalten sein.

Unter räumlicher Zuordnung ist dabei zu verstehen, daß der Farbkuppler sich in einer solchen räumlichen Beziehung zu der betreffenden Silberhalogenidemulsionsschicht befindet, daß eine Wechselwirkung zwischen ihnen möglich ist, die eine bildmäßige Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit der betreffenden lichtempfindlichen Silberhalogenidemulsionsschicht und die Farbe des aus dem räumlich zugeordneten Farbkuppler erzeugten Teilfarbenbildes in einer bestimmten Beziehung zueinander stehen, wobei normalerweise jeder der Spektralempfindlichkeiten (Rot, Grün, Blau) eine andere Farbe des betreffenden Teilfarbenbildes (z.B. Cyan, Magenta, Gelb) zugeordnet ist. Entsprechend der aus den erfindungsgemäßen Farbkupplern gebildeten Farbe (Gelb) werden diese Kuppler vorzugsweise einer Silberhalogenidemulsions-schicht für blaues Licht zugeordnet.

Das Material kann weiterhin von Kupplern verschiedene Verbindungen enthalten, die beispielsweise einen Entwicklungsinhibitor, einen Entwicklungsbeschleuniger, einen Bleichbeschleuniger, einen Entwickler, ein Silberhalogenidlösungsmittel, ein Schleiermittel oder ein Antischleiermittel in Freiheit setzen können, beispielsweise sogenannte DIR-Hydrochinone und andere Verbindungen, wie sie beispielsweise in US-A-4 636 546, 4 345 024, 4 684 604 und in DE-A-31 45 640, 25 15 213, 24 47 079 und in EP-A-198 438 beschrieben sind. Diese Verbindungen erfüllen die gleiche Funktion wie die DIR-, DAR- oder FAR-Kuppler, außer daß sie keine Kupplungsprodukte bilden.

Hochmolekulare Farbkuppler sind beispielsweise in DE-C-1 297 417, DE-A-24 07 569, DE-A-31 48 125, DE-A- 32 17 200, DE-A-33 20 079, DE -A-33 24 932, DE-A- 33 31 743, DE-A-33 40 376, EP-A-27 284, US-A-4 080 211 beschrieben. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Einarbeitung der erfindungsgemäßen Farbkuppler in Silberhalogenidemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittels hängt von der jeweiligen Löslichkeit der Verbindung ab.

Methoden zum Einbringen von in Wasser im wesentlichen unlöslichen Verbindungen durch Mahlverfahren sind beispielsweise in DE-A-26 09 741 und DE-A-26 09 742 beschrieben.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 027, US-A-2 801 170, und EP-A-0 043 037 beschrieben.

Anstelle der hochsiedenden Lösungsmittel können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113.

Die diffusionsfeste Einlagerung anionischer wasserlöslicher Verbindungen (z.B. von Farbstoffen) kann auch mit Hilfe von kationischen Polymeren, sogenannten Beizenpolymeren erfolgen.

Geeignete Ölbildner sind sind z.B. Phthalsäurealkylester, Phosphonsäureester, Phosphorsäureester, Citronensäureester, Benzoesäureester, Amide, Fettsäureester, Trimesinsäureester, Alkohole, Phenole, Anilinderivate und Kohlenwasserstoffe.

Beispiele für geeignete Ölbildner sind Dibutylphthalat, Dicyclohexylphthalat, Di-2-ethylhexylphthalat, Decylphthalat, Triphenylphosphat, Tricresylphosphat, 2-Ethylhexyldiphenylphosphat, Tricyclohexylphosphat, Tri-2-ethylhexylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-t-amylalkohol, Dioctylacetat, Glycerintributyrat, Isostearyllactat, Glycerintributyrat, Trioctylcitrat, N,N-Dibutyl-2-butoxy-5-t-octylanilin, Paraffin, Dodecylbenzol und Diisopropylnaphthalin.

Farbfotografische Negativmaterialien werden üblicherweise durch Entwickeln, Bleichen, Fixieren und Wässern oder durch Entwickeln, Bleichen, Fixieren und Stabilisieren ohne nachfolgende Wässerung verarbeitet, wobei Bleichen und Fixieren zu einem Arbeitsschritt zusammengefaßt sein können. Als Farbentwicklerverbindung lassen sich sämtliche Entwicklerverbindungen verwenden, die die Fähigkeit besitzen, in Form ihres Oxidationsproduktes mit den erfindungsgemäßen Farbkupplern zu Azomethinfarbstoffen zu reagieren. Geeignete Farbentwicklerverbindungen sind aromatische, mindestens eine primäre Aminogruppe enthaltende Verbindungen vom p-Phenylendiamintyp, beispielsweise N,N-Dialkyl-p-phenylendiamine wie N,N-Diethyl-p-phenylendiamin, 1-(N-Ethyl-N-methansulfonamidoethyl)-3-methyl-p-phenylendiamin, 1-(N-Ethyl-N-hydroxyethyl)-3-methyl-p-phenylendiamin und 1-(N-Ethyl-N-methoxyethyl)-3-methyl-p-phenylendiamin. Weitere brauchbare Farbentwickler sind beispielsweise in J. Amer. Chem. Soc. 73, 3106 (1951) und G. Haist, Modern Photographic Processing, 1979, John Wiley and Sons, New York, Seite 545 ff. beschrieben.

Sofern es sich bei den erfindungsgemäßen Farbkupplern um alkalilösliche Verbindungen handelt, können sie auch (anstatt dem fotografischen Material) dem Farbentwickler zugesetzt werden.

Nach der Farbentwicklung kann ein saures Stoppbad oder eine Wässerung folgen. Üblicherweise wird das Material unmittelbar nach der Farbentwicklung gebleicht und fixiert. Als Bleichmittel können z.B. Fe(III)-Salze und Fe(III)-Komplexsalze wie Ferricyanide, Dichromate, wasserlösliche Kobaltkomplexe verwendet werden. Besonders bevorzugt sind Eisen-(III)-Komplexe von Aminopolycarbonsäuren, insbesondere z.B. von Ethylendiamintetraessigsäure, Propylendiamintetraessigsäure, Diethylentriamin-pentaessigsäure, Nitrilotriessigsäure, Imidoessigsäure, N-Hydroxyethylethylendiamintriessigsäure, Alkyliminodicarbonsäure und von entsprechenden Phosphonsäuren. Geeignet als Bleichmittel sind weiterhin Persulfate und Peroxide, z.B. Wasserstoffperoxid.

Auf das Bleichfixierbad oder Fixierbad folgt meist eine Wässerung, die als Gegenstromwässerung ausgeführt ist oder aus mehreren Tanks mit eigener Wasserzufuhr besteht.

Günstige Ergebnisse können bei Verwendung eines darauf folgenden Schlußbades, das keinen oder nur wenig Formaldehyd enthält, erhalten werden.

Die Wässerung kann aber durch ein Stabilisierbad vollständig ersetzt werden, das üblicherweise im Gegenstrom geführt wird. Dieses Stabilisierbad übernimmt bei Formaldehydzusatz auch die Funktion eines Schlußbades.

Bei Farbumkehrmaterialien erfolgt zunächst eine Entwicklung mit einem Schwarz-Weiß-Entwickler, dessen Oxidationsprodukt nicht zur Reaktion mit dem Farbkuppler befähigt ist. Es schließt sich eine diffuse Zweitbelichtung und dann Entwicklung mit einem Farbentwickler, Bleichen und Fixieren an.

### Beispiel

Gelb-Einzelgüsse für ein zur Schnellverarbeitung geeignetes farbfotografisches Material werden hergestellt, indem auf eine Seite eines Schichttägers aus beiderseits mit Polyethylen kaschiertem Papier jeweils 4 Schichten in der angegebenen Reihenfolge aufgetragen werden.

Die Gewichtsangaben beziehen sich jeweils auf 1 m².

Für den Silberhalogenidauftrag werden die entsprechenden Mengen AgNO₃ angegeben.

### Prüfmaterial 1A (Vergleich)

| | |
|---|---|
| Schicht 1 | Substrierschicht mit 0,2 g Gelatine |
| | |
| Schicht 2 | blauempfindliche Silberhalogenidemulsionsschicht |
| | (99,5 mol-% AgCl und 0,5 mol-% AgBr; mittlerer Korndurchmesser |
| | 0,8 pm)aus 0,46 g AgNO₃ |
| | 1,3 g Gelatine |
| | 0,5 g Gelbkuppler XY-1 |
| | 0,2 g Weißkuppler XW-1 |
| | 0,5 g Polyester aus Adipinsäure, Butan-1,3-diol und Hexa-1,6-diol |
| | |
| Schicht 3 | 1,1 g Gelatine |
| | 0,06 g Dioctylhydrochinon |
| | 0,06 g Di-n-butylphthalat |
| | |
| Schicht 4 | 0.9 g Gelatine |
| | 0,3 g Soforthärtungsmittel XH-1 |

Im Prüfmaterial 1A wurden folgende Verbindungen verwendet.

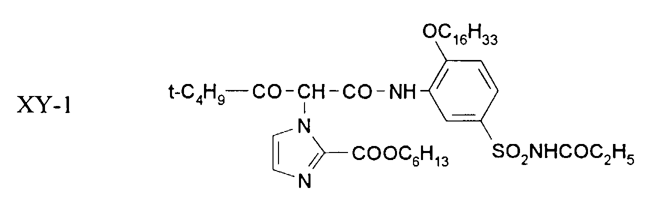

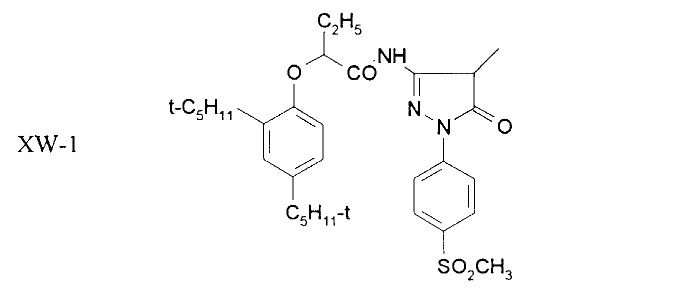

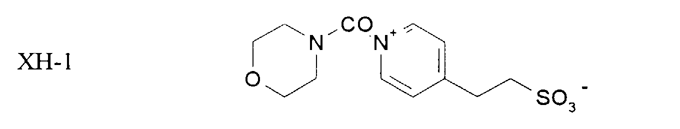

### Prüfmaterial 1B (gemäß Erfindung)

Wie Prüfmaterial 1A, jedoch mit folgender Änderung:

Schicht 2 enthält anstelle des Vergleichsgelbkupplers XY-1 0,58 g des erfindungsgemäßen Kupplers Y-1.

Die erhaltenen Prüfmaterialien wurden hinter einem Graukeil mit blauem Licht belichtet und wie nachfolgend angegeben verarbeitet.

| | | |
|---|---|---|
| a) | Farbentwicklung 45 s | 35 °C |
| | Triethanolamin | 9,0 g |
| | N,N-Diethylhydroxylamin | 6,0 g |
| | Diethylenglykol | 0,05 g |
| | 3-Methyl-4-amino-N-ethyl-N-methansulfonamidoethylanilin-sulfat | 6,0 g |
| | Kaliumsulfat | 0,2 g |
| | Triethylenglykol | 0,05 g |
| | Kalimcarbonat | 22,0 g |
| | Kaliumhydroxid | 0,4 g |
| | Ethylendiamintetraessigsäure | 2,2 g |
| | | |
| | auffüllen mit Wasser auf 1.000 ml; pH 9,2 | |
| | | |
| b) | Bleichfixierbad 45 s | 35 °C |
| | Ammoniumthiosulfat | 75,0 g |
| | Natriumhydrogensulfit | 13,5 g |
| | Ammoniumacetat | 2,0 g |
| | Ethylendiamintetraessigsäure | |
| | (Eisen-Ammonium-Salz) | 57,0g |
| | Ammoniak 25 %ig | 9,5 g |
| | Essigsäure | 9,0 g |
| | | |
| | auffüllen mit wasser auf 1.000 ml; pH 5,5 | |
| | | |
| c) | Wässern 2 min | 33 °C |

Man erhält sowohl für Schichtaufbau 1 A (Vergleich) wie für Schichtaufbau 1 B (Erfindung) je einen gelben Farbkeil. Die Maximaldichten betragen 2,2 bzw. 2,08 gemessen hinter Blaufilter. Der mit dem erfindungsgemäßen Material erhaltene Farbkeil zeichnet sich durch hohe Farbreinheit bzw. Brillanz aus.

Dieses Beispiel belegt die Eignung der erfindungsgemäßen Verbindungen als Gelbkuppler.

## Patentansprüche

1. Fotografisches Farbentwicklungsverfahren, bei dem ein farbfotografisches Aufzeichnungsmaterial mit mindestens einer bildmäßig belichteten Silberhalogenidemulsionsschicht in Gegenwart einer Farbkupplerverbindung und einer Farbentwicklerverbindung entwickelt wird, dadurch gekennzeichnet, daß die Farbkupplerverbindung der Formel I entspricht 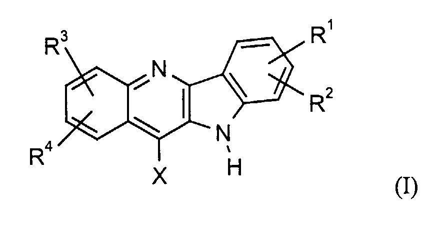 worin
R¹ ,R², R³, R⁴ ein H-Atom oder einen Substituenten;
X ein H-Atom oder einen Rest, der unter den Bedingungen der chromogenen Entwicklung abgespalten wird bedeuten.

2. Farbfotografisches Aufzeichnungsmaterial mit einem Schichtträger und mindestens einer darauf angeordneten lichtempfindlichen Silberhalogenidemulsionsschicht, der ein Farbkuppler zugeordnet ist, dadurch gekennzeichnet, daß der Farbkuppler der folgenden allgemeinen Formel I entspricht 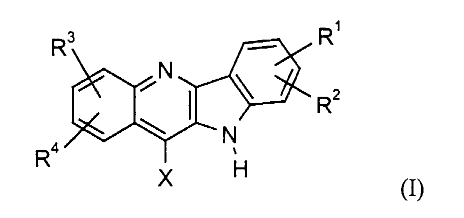 worin
R¹, R², R³, R⁴ ein H-Atom oder einen Substituenten;
X ein H-Atom oder einen Rest, der unter den Bedingungen der chromogenen Entwicklung abgespalten wird bedeuten.

3. Aufzeichnungsmaterial nach Anspruch 2, dadurch gekennzeichnet. daß der Farbkuppler der Formel II entspricht 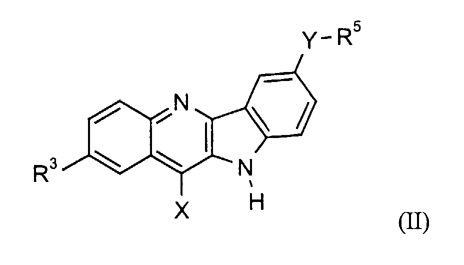 worin
R³ H oder Alkyl;
R⁵ eine gegebenenfalls substituierte Alkylgruppe;
X H oder einen unter den Bedingungen der chromogenen Entwicklung abspaltbaren Rest;
Y -NH-CO- oder -NH-SO₂- bedeuten.

## Claims

1. Photographic colour development process in which a colour-photographic recording material having at least one imagewise-exposed silver-halide emulsion layer is developed in the presence of a colour coupler compound and a colour developer compound, characterized in that the colour coupler compound conforms to the formula I 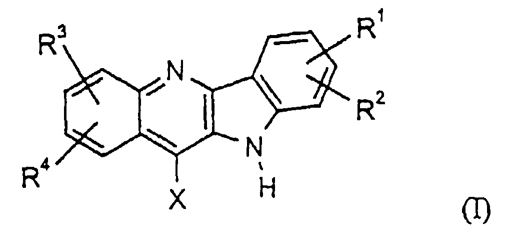 in which
R¹, R², R³ and R⁴ are an H atom or a substituent;
X is an H atom or a radical which is cleaved off under the conditions of chromogenic development.

2. Colour-photographic recording material having a layer support and at least one photosensitive silver-halide emulsion layer arranged thereon which is assigned to a colour coupler, characterized in that the colour coupler conforms to the following general formula I 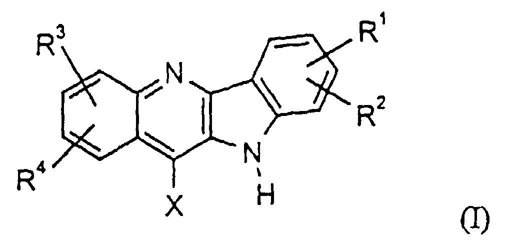 in which
R¹, R², R³ and R⁴ are an H atom or a substituent;
X is an H atom or a radical which is cleaved off under the conditions of chromogenic development.

3. Recording material according to Claim 2, characterized in that the colour coupler conforms to the formula II 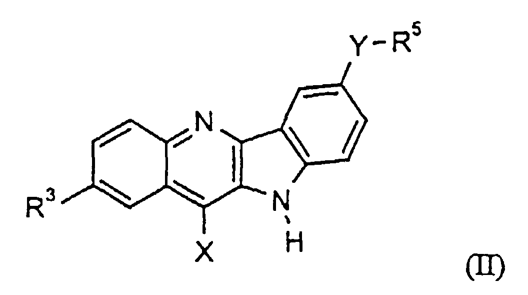 in which
R³ is H or alkyl;
R⁵ is an optionally substituted alkyl group;
X is H or a radical which can be cleaved off under the conditions of chromogenic development;
Y is -NH-CO- or -NH-SO₂-.

## Revendications

1. Procédé photographique de développement chromogène dans lequel on développe un matériau d'enregistrement pour photographie en couleurs comprenant au moins une couche d'émulsion à l'halogénure d'argent exposée en forme d'image en présence d'un composé de copulant chromogène et d'un composé de révélateur chromogène, caractérisé en ce que le composé de copulant chromogène répond à la formule I 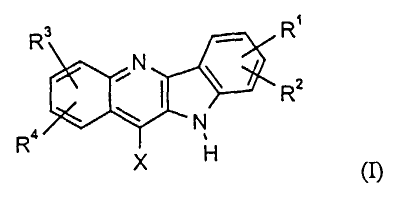 dans laquelle
R¹, R², R³, R⁴ représentent un atome d'hydrogène ou un substituant;
X représente un atome d'hydrogène ou un radical qui se sépare dans les conditions du développement chromogène.

2. Matériau d'enregistrement pour la photographie en couleur comprenant un support de couche et au moins une couche photosensible d'émulsion à l'halogénure d'argent disposée sur cette dernière, à laquelle est attribué un copulant chromogène, caractérisé en ce que le copulant chromogène répond à la formule générale I ci-après 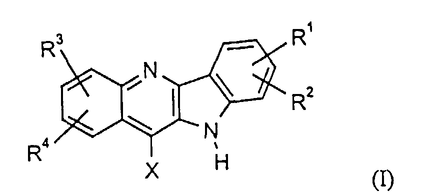 dans laquelle
R¹, R², R³, R⁴ représentent un atome d'hydrogène ou un substituant;
X représente un atome d'hydrogène ou un radical qui se sépare dans les conditions du développement chromogène.

3. Matériau d'enregistrement selon la revendication 2, caractérisé en ce que le copulant chromogène répond à la formule II 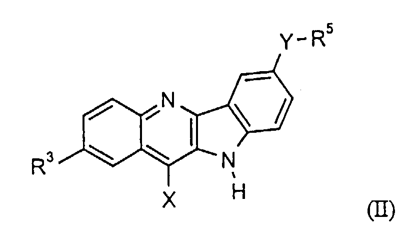 dans laquelle
R³ représente un atome d'hydrogène ou un groupe alkyle;
R⁵ représente un groupe alkyle le cas échéant substitué;
X représente un atome d'hydrogène ou un radical qui se sépare dans les conditions du développement chromogène;
Y représente un groupe -NH-CO- ou un groupe -NH-SO₂.
